# EUROPEAN PATENT APPLICATION

(11) **EP 4 582 135 A1**
(43) Date of publication of application: **09.07.2025**
(21) Application number: 23888365.6
(22) Date of filing: 22.09.2023
(51) Int. Cl.: A61N 1/08, A61N 1/36

(54) **PULSED RADIOFREQUENCY TREATMENT DEVICE AND PULSED RADIOFREQUENCY TREATMENT SYSTEM**

(30) Priority: 08.11.2022 JP 2022178996
(71) Applicant: Kabushiki Kaisha Top, Adachi-ku, Tokyo 120-0035 (JP)
(72) Inventor: IKEUCHI Atsushi, Tokyo 120-0035 (JP)
(74) Representative: Schön, Christoph
(86) International application number: PCT/JP2023/034431
(87) International publication number: WO 2024/101012

(57) **Abstract**

Provided is a pulsed radiofrequency treatment device and the like capable of reducing the burden on a person to be treated and improving convenience when using an electrode pad. A pulsed radiofrequency treatment device 10 includes a pulse generation unit 11e, a device display 11c, a device storage unit 11b, a device control unit 11a, and the like. When a treatment start operation is performed by a patient P operating the device display 11c, the device control unit 11a controls the output state of a radiofrequency pulse current by the pulse generation unit 11e based on setting information stored in the device storage unit 11b.

## Description

### Technical Field

The present invention relates to a pulsed radiofrequency treatment device and the like to perform pulsed radiofrequency treatment.

### Background Art

In recent years, a pulsed radiofrequency treatment method using a pulsed radiofrequency treatment device has been implemented as a method of treating pain caused by spinal disease, neurological disorder, and the like (for example, Patent Literature 1). Unlike a method of injecting local anesthetic such as a nerve block, this pulsed radiofrequency treatment method is such that a user operates the pulsed radiofrequency treatment device to generate an electric field in an affected area of a person to be treated in order to obtain an analgesic effect.

Further, as pulsed radiofrequency treatment devices, there are a type of pulsed radiofrequency treatment device using an electrode pad, a type of pulsed radiofrequency treatment device using an electrode needle, and the like to generate an electric field. Here, the pulsed radiofrequency treatment device using the electrode pad has the advantage that invasiveness is low compared to the pulsed radiofrequency treatment device using the electrode needle, and nerve block.

### Citation List

### Patent Literature

Patent Literature 1: Published Japanese Translation of PCT application No. 2013-508119

### Summary of Invention

### Technical Problem

In the pulsed radiofrequency treatment device using the electrode pad mentioned above, when a person to be treated such as a patient uses the pulsed radiofrequency treatment device while operating it himself or herself, there is a possibility that an optimum treatment effect cannot be obtained, compared to a case where a therapist such as a doctor performs treatment by operating the pulsed radiofrequency treatment device while observing the progress of the treatment of a treated person. However, when the person to be treated tries to receive the treatment using the pulsed radiofrequency treatment device by a therapist's operation, the person to be treated needs to go to a medical institution where the therapist works, and hence there is a problem that the burden on the patient is heavy and convenience is low.

The present invention has been made to solve the above problem, and it is an object thereof to provide a pulsed radiofrequency treatment device and the like capable of reducing the burden on a person to be treated and improving convenience when using an electrode pad.

### Solution to Problem

In order to achieve the above object, a pulsed radiofrequency treatment device according to Claim 1 including: a pulse generation unit which outputs a radiofrequency pulse current for giving pulsed radiofrequency treatment to a person to be treated; an electrode pad connected to the pulse generation unit to supply the radiofrequency pulse current to an affected area; a communication unit which receives, through a communication network, setting information including a set value of the radiofrequency pulse current; a storage unit which stores the setting information received by the communication unit; an operation unit for accepting a treatment start operation; and a control unit which controls an output state of the radiofrequency pulse current by the pulse generation unit based on the setting information stored in the storage unit when the operation unit accepts the treatment start operation.

According to this pulsed radiofrequency treatment device, the setting information including the set value of the radiofrequency pulse current is received by the communication unit through the communication network, and stored in the storage unit. Then, when the operation unit accepts the treatment start operation, the output state of the radiofrequency pulse current by the pulse generation unit is controlled by the control unit based on the setting information stored in the storage unit.

As described above, since the setting information for controlling the output state of the radiofrequency pulse current is received by the communication unit of the pulsed radiofrequency treatment device through the communication network, the person to be treated can receive pulsed radiofrequency treatment, from which the optimum treatment effect can be obtained, at home or the like without going to a medical institution. Therefore, the burden on the treated person can be reduced, and hence convenience can be improved.

The invention according to Claim 2 is the pulsed radiofrequency treatment device according to Claim 1 further including an output interface which outputs information, wherein the electrode pad is composed of electrode pads with a plurality of sizes detachable from the pulse generation unit, the setting information further includes information on a set size of the electrode pad in addition to the set value of the radiofrequency pulse current, and the control unit controls the output interface to output error information representing a discrepancy between a size of the electrode pad connected to the pulse generation unit and the set size when the size does not match the set size in the setting information, and even when the treatment start operation is performed, the control unit stops output of the radiofrequency pulse current from the pulse generation unit.

According to this pulsed radiofrequency treatment device, when the size of the electrode pad connected to the pulse generation unit does not match the set size in the setting information, the output interface is controlled to output the error information representing the discrepancy between the size and the set size, and even when the treatment start operation is performed, output of the radiofrequency pulse current from the pulse generation unit is stopped. Therefore, when the size of the electrode pad mounted onto the person to be treated is wrong, a first user or the person to be treated can recognize that effect. For the same reason, it can avoid performing pulsed radiofrequency treatment in such a state that the electrode pad with the wrong size is used.

A pulsed radiofrequency treatment system according to Claim 3 including: the pulsed radiofrequency treatment device according to Claim 1 or 2; a server having a server storage unit for storing a database; a first medical terminal configured to be able to communicate with the server to set the setting information; and a user terminal configured to be able to communicate with the communication unit of the pulsed radiofrequency treatment device and the server, wherein at the time when one pulsed radiofrequency treatment is completed, the control unit controls the communication unit to transmit, to the user terminal, treatment implementation information representing implementation status of the pulsed radiofrequency treatment, the user terminal is configured to be able to transmit, to the server, the treatment implementation information received from the communication unit, the server is configured to transmit, to the user terminal, the setting information when receiving the setting information from the first medical terminal, and to store the setting information and the treatment implementation information from the user terminal in the server storage unit as the database, and the first medical terminal is configured to be accessible to the database stored in the server storage unit by communicating with the server.

According to this pulsed radiofrequency treatment system, the setting information is set on the first medical terminal, and when the setting information is received from the first medical terminal by the server, the setting information is transmitted to the user terminal. Further, at the time when one pulsed radiofrequency treatment is completed, the treatment implementation information representing the implementation status of the pulsed radiofrequency treatment is transmitted from the communication unit to the user terminal, and hence the treatment implementation information is transmitted from the user terminal to the server. Then, inside the server, the setting information from the first medical terminal and the treatment implementation information from the user terminal are stored in the server storage unit as the database. Thus, the database obtained by turning the setting information and implementation information on the pulsed radiofrequency treatment into data in chronological order can be created inside the server. Further, the therapist can access the database through the first medical terminal to grasp the progress of the pulsed radiofrequency treatment for the treated person.

The invention according to Claim 4 is the pulsed radiofrequency treatment system according to Claim 3 in which the setting information includes information on a mounting position of the electrode pad onto the person to be treated in addition to the set value of the radiofrequency pulse current, and the user terminal has a terminal output interface to output the information included in the setting information on the mounting position of the electrode pad onto the person to be treated when receiving the setting information from the server.

According to this pulsed radiofrequency treatment system, when receiving the setting information from the server by the user terminal, the information on the mounting position of the electrode pad onto the person to be treated is output from the terminal output interface. Thus, the person to be treated or the like can recognize the mounting position of the electrode pad onto the person to be treated, and hence the electrode pad can be mounted in a proper position of the person to be treated.

The invention according to Claim 5 is the pulsed radiofrequency treatment system according to Claim 4 further including a second medical terminal configured to be able to reference data other than information that identifies the person to be treated from the database stored in the server storage unit by communicating with the server.

According to this pulsed radiofrequency treatment system, any medical worker other than the therapist can use the second medical terminal to reference data other than the information that identifies the person to be treated from the database stored in the server storage unit. Thus, the medical worker can improve the knowledge of the pulsed radiofrequency treatment, and hence can use the data as a reference when the medical worker himself or herself performs the pulsed radiofrequency treatment.

### Brief Description of Drawings

FIG. 1 is a diagram illustrating the configuration of a pulsed radiofrequency treatment system including a pulsed radiofrequency treatment device according to one embodiment of the present invention.
FIG. 2 is a block diagram illustrating an electrical configuration of a pulse generator.
FIG. 3 is a block diagram illustrating an electrical configuration of a user terminal.
FIG. 4 is a block diagram illustrating an electrical configuration of a server.
FIG. 5 is a sequence diagram illustrating a control operation example between an attending doctor terminal and the server.
FIG. 6 is a sequence diagram illustrating a control operation example among the pulsed radiofrequency treatment device, the user terminal, and the server.
FIG. 7 is a flowchart illustrating control processing of pulsed radiofrequency treatment in the pulse generator.
FIG. 8 is a sequence diagram illustrating another control operation example among the pulsed radiofrequency treatment device, the user terminal, and the server.
FIG. 9 is a sequence diagram illustrating a control operation example between a medical terminal and the server.

### Description of Embodiment

A pulsed radiofrequency treatment device and a pulsed radiofrequency treatment system according to one embodiment of the present invention will be described with reference to the drawings. A pulsed radiofrequency treatment system 1 uses a pulsed radiofrequency treatment device 10 to give pulsed radiofrequency treatment to a patient P (person to be treated).

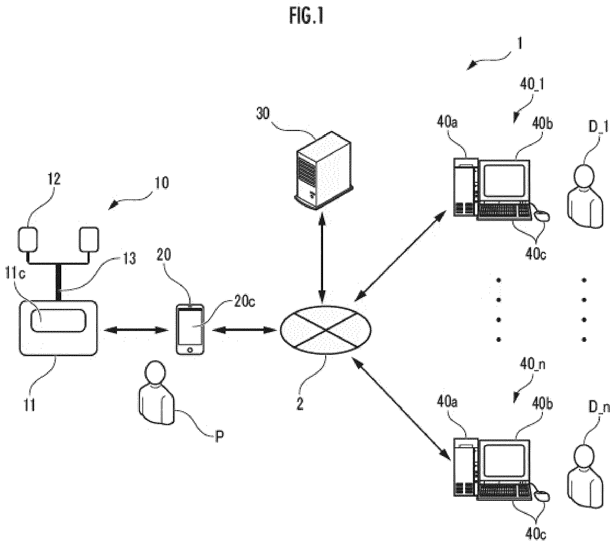

As illustrated in FIG. 1, the pulsed radiofrequency treatment system 1 includes the pulsed radiofrequency treatment device 10, a user terminal 20, a server 30, and n (n is plural) number of medical terminals 40_1 to 40_n (only two medical terminals are illustrated).

In this pulsed radiofrequency treatment system 1, the user terminal 20, the server 30, and the n number of medical terminals 40_1 to 40_n are communicably connected to one another through a communication network 2, and this communication network 2 is composed of an internet communication network, a LAN communication network, and the like.

Note that only one pulsed radiofrequency treatment device 10 and one user terminal 20 are illustrated in FIG. 1 for ease of understanding, but plural pulsed radiofrequency treatment devices 10 and plural user terminals 20 can be connected to the communication network 2 in the case of this pulsed radiofrequency treatment system 1.

In this pulsed radiofrequency treatment system 1, any user other than a patient P can operate the pulsed radiofrequency treatment device 10 and/or the user terminal 20, but such a case that the pulsed radiofrequency treatment device 10 and the user terminal 20 are operated by the patient P will be described in the present embodiment by taking the case as an example.

First, the pulsed radiofrequency treatment device 10 will be described. This pulsed radiofrequency treatment device 10 is to give pulsed radiofrequency treatment to the patient P, which includes a pulse generator 11 and each electrode pad 12.

The electrode pad 12 has a pad shape with an electrode built therein, and plural sizes of electrode pads 12 (only one size is illustrated) are provided. The electrode pad 12 is connected to the pulse generator 11 in a detachable state through a cable 13 upon pulsed radiofrequency treatment. Further, an adhesive gel is placed on the surface of the electrode pad 12, and the electrode pad 12 is attached to an affected area of the patient P due to the stickiness of the adhesive gel upon pulsed radiofrequency treatment.

In this pulsed radiofrequency treatment device 10, an electric field is generated in the affected area of the patient P by supplying a radiofrequency pulse current from the pulse generator 11 to the electrode pad 12 in such a state that the electrode pad 12 is attached to the affected area of the patient P. Therefore, the patient P can obtain an analgesic effect in the affected area.

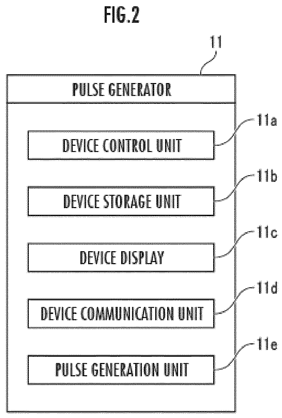

The pulse generator 11 is to generate the radiofrequency pulse current for pulsed radiofrequency treatment, and includes a device control unit 11a, a device storage unit 11b, a device display 11c, a device communication unit 11d, and a pulse generation unit 11e as illustrated in FIG. 2.

Note that, in the present embodiment, the device control unit 11a corresponds to a control unit, the device storage unit 11b corresponds to a storage unit, the device display 11c corresponds to an operation unit and an output interface, and the device communication unit 11d corresponds to a communication unit.

The device control unit 11a is configured by a CPU, memories, an I/O interface, and the like to control the operating states and the like of the device storage unit 11b, the device display 11c, the device communication unit 11d, and the pulse generation unit 11e. Further, the device control unit 11a is configured to recognize the size of the electrode pad 12 connected to the pulse generator 11.

The device storage unit 11b is configured by a storage device such as a RAM. When a signal from the user terminal 20 is received by the device communication unit 11d, data contained in the signal is stored in this device storage unit 11b.

For example, as will be described later, when a setting information signal from the user terminal 20 is received by the device communication unit 11d, setting information contained in the setting information signal is stored. In this setting information, information on the set size and mounting position of the electrode pad 12, and output setting information (supply time, pulse frequency, pulse width, voltage, and the like) of the radiofrequency pulse current in the pulse generation unit 11e.

Further, when authentication operation is executed to perform near field communication (for example, Bluetooth: registered trademark) with the user terminal 20, the authentication data is stored in the device storage unit 11b.

The device display 11c is configured by a capacitive touch panel, and placed on the front of the pulse generator 11. When the setting information signal is received by the device communication unit 11d, the device control unit 11a controls the device display 11c to display, on the device display 11c, the set size and mounting position of the electrode pad 12 contained in the setting information signal.

Further, the device control unit 11a is configured to recognize the operating sate of the device display 11c by the patient P.

The device communication unit 11d is configured by a communication device to execute near field communication with a terminal communication unit 20d of the user terminal 20 to be described later.

The pulse generation unit 11e is configured by an electric circuit, which is controlled by the device control unit 11a when performing the pulsed radiofrequency treatment to generate a radiofrequency pulse current corresponding to the output setting information on the radiofrequency pulse current described above and to supply the generated radiofrequency pulse current to the electrode pad 12.

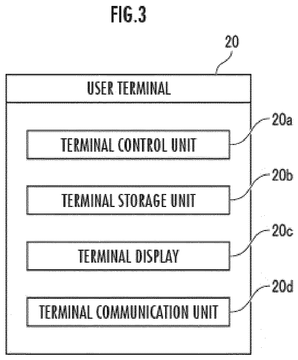

Next, the user terminal 20 will be described. The user terminal 20 is a type of smartphone, and includes a terminal control unit 20a, a terminal storage unit 20b, a terminal display 20c, and a terminal communication unit 20d as illustrated in FIG. 3. Note that the terminal display 20c corresponds to a terminal output interface in the present embodiment.

The terminal control unit 20a is configured by a CPU, memories, an I/O interface, and the like to control the operating states of the terminal storage unit 20b, the terminal display 20c, and the terminal communication unit 20d.

The terminal storage unit 20b is configured by storage devices such as a storage, a ROM, a RAM, and the like. When signals from the server 30 and the pulsed radiofrequency treatment device 10 are received by the terminal communication unit 20d, various data contained in the signals are stored in this terminal storage unit 20b.

For example, as will be described later, when the above-described setting information signal from the server 30 is received by the terminal communication unit 20d, the mounting position and set size of the electrode pad 12 in the setting information contained in the setting information signal are stored. Further, in the terminal storage unit 20b, application software for pulsed radiofrequency treatment to perform the pulsed radiofrequency treatment (hereinafter called "treatment software") is installed.

The terminal display 20c is configured by a capacitive touch panel and placed over most of the front of the user terminal 20. The display state of the terminal display 20c is controlled by the terminal control unit 20a. For example, as will be described later, the mounting position and set size of the electrode pad 12 are displayed on the terminal display 20c in such a state that the treatment software is started up. Further, the terminal control unit 20a is configured to recognize the operating state of the terminal display 20c by the patient P.

The terminal communication unit 20d is configured by a communication device to execute near field communication with the device communication unit 11d of the pulse generator 11 and to execute wireless communication with the server 30 through the communication network 2.

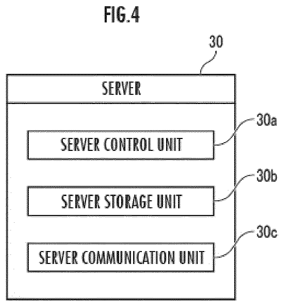

Next, the server 30 will be described. As illustrated in FIG. 4, the server 30 includes a server control unit 30a, a server storage unit 30b, and a server communication unit 30c. The server control unit 30a is configured by a CPU, memories, an I/O interface, and the like to control the operating states of the server storage unit 30b and the server communication unit 30c.

The server communication unit 30c is configured by a communication device to execute communication between the user terminal 20 and n medical terminals 40_1 to 40_n through the communication network 2 in a manner to be described later.

The server storage unit 30b is configured by storage devices such as a ROM, a RAM, an HDD, and the like. As will be described later, when the above-described setting information signal is received by the server communication unit 30c by communicating with the medical terminal 40_1, the server storage unit 30b stores setting information contained in the setting information signal as a database of the patient P.

Further, by communication with the user terminal 20, when a treatment implementation information signal to be described later is received by the server communication unit 30c, the server storage unit 30b stores treatment implementation information contained in the treatment implementation information signal as the database of the patient P. In addition to this, when a treatment effect information signal to be described later is received by the server communication unit 30c, the server storage unit 30b stores treatment effect information on the patient P contained in the treatment effect information signal as the database of the patient P.

In this case, when the treatment effect information is included in the database of the patient P, a patient ID of the patient P, setting information, treatment implementation information, and treatment effect information are created in association with one another, while when no treatment effect information is included, the patient ID of the patient P, setting information, and treatment implementation information are created in association with one another.

Next, the n medical terminals 40_1 to 40_n will be described. Each of the n medical terminals 40_1 to 40_n is of a type of desktop personal computer used by each of doctors D_1 to D_n, respectively. Note that in the following description, the n medical terminals 40_1 to 40_n are collectively called the "medical terminal 40" as appropriate, and the doctors D_1 to D_n are collectively called the "doctor D" as appropriate.

As illustrated in FIG. 1, the medical terminal 40 includes a terminal body 40a, a display 40b, an input interface 40c, and the like. In this body 40a, a microcomputer, a storage, and a communication device (none of which are illustrated) are built. The microcomputer is configured by a CPU, memories, an I/O interface, and the like, and the storage is configured by an HDD or an SSD.

In this storage, a communication management program for executing communication with the server 30 is stored, and data received by the microcomputer through the communication device, authentication data for communication with the server 30, and the like are stored.

The input interface 40c is configured by a keyboard, a mouse, and the like to operate the medical terminal 40. In this medical terminal 40, the doctor D operates the input interface 40c to run the communication management program so as to execute communication with the server 30 in a manner to be described later.

Note that in the following description, the doctor D_1 in charge of treating the patient P is called the "attending doctor D_1" as appropriate, and the medical terminal 40_1 operated by the attending doctor D_1 is called the "attending doctor terminal 40_1" as appropriate. Further, in the present embodiment, the attending doctor D_1 corresponds to a therapist, the attending doctor terminal 40_1 corresponds to a first medical terminal, and the medical terminal 40_n corresponds to a second medical terminal.

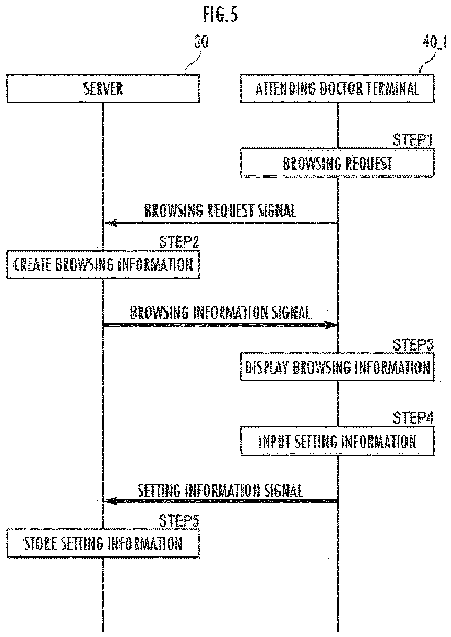

Referring next to FIG. 5, control processing operation executed between the attending doctor terminal 40_1 and the server 30 in the pulsed radiofrequency treatment system will be described.

Here, the control processing operation illustrated in FIG. 5 is such that the attending doctor D_1 transmits setting information to the server 30 through the attending doctor terminal 40_1, which is executed between the attending doctor terminal 40_1 and the server 30 in a state where authentication operation of the attending doctor terminal 40_1 is completed.

As illustrated in FIG. 5, browsing request processing is first executed on the attending doctor terminal 40_1 (FIG. 5/STEP1). In this browsing processing, a patient ID is input by the attending doctor D_1 operating the input interface 40c in such a state that browsing software (for example, a browser) is started up on the attending doctor terminal 40_1. Thus, a browsing request signal containing the patient ID of the patient P is transmitted from the attending doctor terminal 40_1 to the server 30.

When this browsing request signal is received by the server 30, browsing information creation processing is executed on the server 30 (FIG. 5/STEP2). In this browsing information creation processing, the database of the patient P inside the server 30 is referred to based on the patient ID contained in the browsing request signal to create browsing information.

This browsing information is created to include progress information on the pulsed radiofrequency treatment given to the patient P. In this progress information, the treatment implementation information and the setting information described above are included, and in addition to these, treatment effect information to be described later may also be included. When the browsing information is created in this way, the browsing information signal containing this browsing information is transmitted from the server 30 to the attending doctor terminal 40_1.

When this browsing information signal is received by the attending doctor terminal 40_1, browsing information display processing is executed on the attending doctor terminal 40_1 (FIG. 5/STEP3). In this browsing information display processing, the browsing information contained in the browsing information signal is displayed on the display 40b of the attending doctor terminal 40_1.

The attending doctor D_1 refers to this browsing information to decide on a plan for the next pulsed radiofrequency treatment to be given to the patient P based on the progress information on pulsed radiofrequency treatments given to the patient P so far. In other words, information on the set size and mounting position of the electrode pad 12 and output setting information (supply time, pulse frequency, pulse width, voltage, and the like) of the radiofrequency pulse current in the pulse generation unit 11e are determined as setting information.

Next, setting information input processing is executed (FIG. 5/STEP4). In this setting information input processing, the attending doctor D_1 operates the input interface 40c of the attending doctor terminal 40_1 to input the setting information determined as mentioned above. Along with that, a setting information signal containing this setting information is transmitted from the attending doctor terminal 40_1 to the server 30.

When this setting information signal is received by the server 30, setting information storage processing is executed on the server 30 (FIG. 5/STEP5). In this setting information storage processing, the setting information contained in the setting information signal is stored in the server storage unit 30b as the database of the patient P.

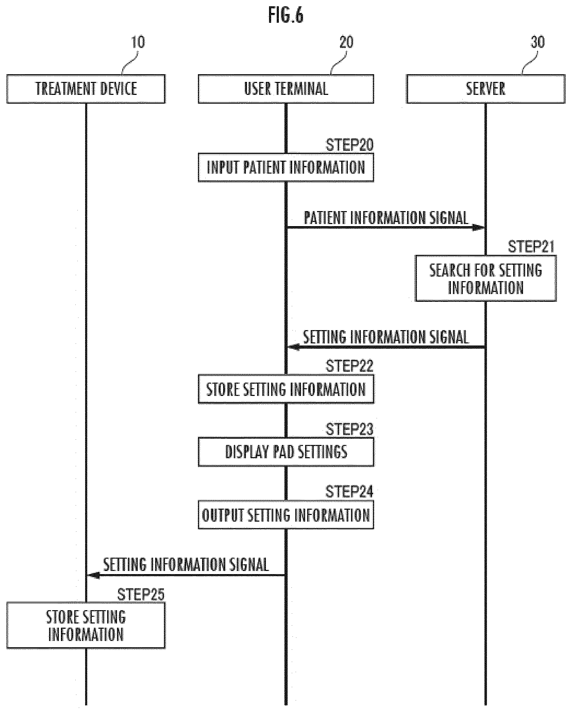

Referring next to FIG. 6, control processing operation executed among the server 30, the user terminal 20, and the pulsed radiofrequency treatment device 10 (written as "treatment device" in FIG. 6) in the pulsed radiofrequency treatment system will be described.

Here, the control processing operation illustrated in FIG. 6 corresponds to preparation processing for the patient P to receive pulsed radiofrequency treatment, which is executed in a state where the authentication operation of the user terminal 20 is completed between the user terminal 20 and the server 30, and authentication operations of both of the user terminal 20 and the pulsed radiofrequency treatment device 10 are completed therebetween.

As illustrated in FIG. 6, patient information input processing is executed on the user terminal 20 (FIG. 6/STEP20). In this patient information input processing, the patient P operates the user terminal 20 to enter the patient ID of the patient P in such a state that the treatment software is started up. When the patient information input processing is executed in this way, a patient information signal containing the patient ID is transmitted from the user terminal 20 to the server 30.

When this patient information signal is received by the server 30, setting information search processing is executed on the server 30 (FIG. 6/STEP21). In this setting information search processing, the database inside the server 30 is searched for setting information based on the patient ID contained in the patient information signal. As described above, this setting information is various information on the pulsed radiofrequency treatment set by the attending doctor D_1.

When the setting information search processing is executed in this way, the setting information signal containing the setting information searched for as mentioned above is transmitted from the server 30 to the user terminal 20.

When this setting information signal is received by the user terminal 20, setting information storage processing is executed on the user terminal 20 (FIG. 6/STEP22). In this setting information storage processing, information on the set size and mounting position of the electrode pad 12 in the setting information contained in the setting information signal is stored in the terminal storage unit 20b.

Next, pad setting display processing is executed (FIG. 6/STEP23). In this pad setting display processing, the information on the set size and mounting position of the electrode pad 12 is displayed on the terminal display 20c.

Next, setting information output processing is executed (FIG. 6/STEP24). In this setting information output processing, the setting information signal containing the setting information described above is transmitted from the user terminal 20 to the pulsed radiofrequency treatment device 10. Note that in this case, it may be so configured that a signal containing any information other than the information on the mounting position of the electrode pad 12 is transmitted as setting information to the pulsed radiofrequency treatment device 10.

When this setting information signal is received by the pulsed radiofrequency treatment device 10, setting information storage processing is executed (FIG. 6/STEP25). In this setting information storage processing, the setting information contained in the setting information signal, that is, the information on the set size and mounting position of the electrode pad 12 and output setting information of the radiofrequency pulse current is stored in the device storage unit 11b of the pulse generator 11.

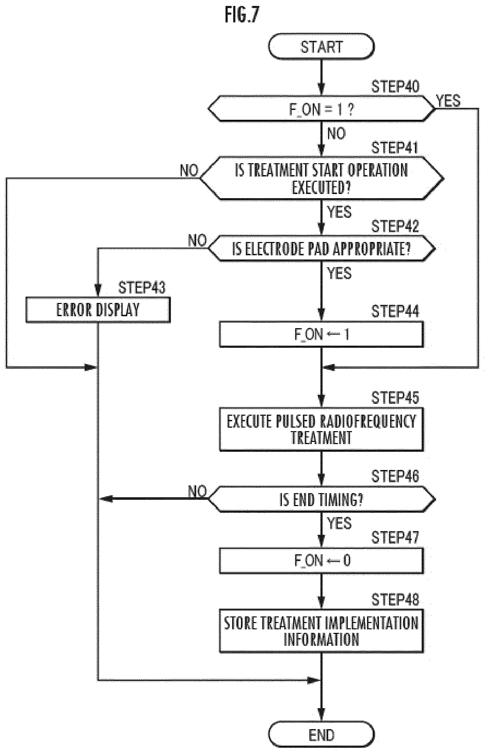

Referring next to FIG. 7, control processing operation upon pulsed radiofrequency treatment executed by the pulse generator 11 of the pulsed radiofrequency treatment device 10 will be described.

As illustrated in FIG. 7, it is first determined whether or not an under treatment flag F_ON is "1" (FIG. 7/STEP40). This under treatment flag F_ON indicates whether or not the pulsed radiofrequency treatment is in progress, and the value is set in a manner to be described later, and set to "0" when the pulse generator 11 is started up.

When this determination is negative (FIG. 7/STEP40...NO) and the pulsed radiofrequency treatment is not in progress, it is determined whether or not a treatment start operation is executed (FIG. 7/STEP41). In this case, when a start button (not illustrated) being displayed on the device display 11c of the pulse generator 11 is pressed down, it is determined that the treatment start operation is executed, while in the other cases, it is determined that the treatment start operation is not executed.

When this determination is negative (FIG. 7/STEP41...NO) and the treatment start operation is not executed, this processing is ended as it is.

On the other hand, when this determination is positive (FIG. 7/STEP41... YES) and the treatment start operation is executed, it is determined whether or not the electrode pad 12 is appropriate (FIG. 7/STEP42). In this case, when the size of the electrode pad 12 attached to the pulse generator 11 is the same as the set size in the setting information, it is determined that the electrode pad 12 is appropriate, while in the other cases, it is determined that the electrode pad 12 is inappropriate. For example, even when the electrode pad 12 is not attached to the pulse generator 11, it is determined that the electrode pad 12 is inappropriate.

When this determination is negative (FIG. 7/STEP42...NO) and the electrode pad 12 is inappropriate, error display processing is executed (FIG. 7/STEP43). In this error display processing, an error message saying "an appropriate electrode pad is not attached" is displayed on the device display 11c of the pulse generator 11 (not illustrated). After that, this processing is ended.

On the other hand, the determination mentioned above is positive (FIG. 7/STEP42.. YES) and the electrode pad 12 is appropriate, the under treatment flag F_ON is set to "1" to represent that the pulsed radiofrequency treatment is in progress (FIG. 7/STEP44).

Next, the pulsed radiofrequency treatment is executed (FIG. 7/STEP45). Specifically, the radiofrequency pulse current is supplied from the pulse generator 11 to the electrode pad 12 based on the setting information (supply time, pulse frequency, pulse width, voltage, and the like) of the radiofrequency pulse current stored in the device storage unit 11b.

Next, it is determined whether or not it is the end timing of the pulsed radiofrequency treatment (FIG. 7/STEP46). When this determination is negative (FIG. 7/STEP46...NO) and it is not the end timing of the pulsed radiofrequency treatment, this processing is ended as it is.

On the other hand, when this determination is positive (FIG. 7/STEP46... YES) and it is the end timing of the pulsed radiofrequency treatment, the under treatment flag F_ON is set to "0" to represent that fact (FIG. 7/STEP47).

Next, treatment implementation information storage processing is executed (FIG. 7/STEP48). In this treatment implementation information storage processing, treatment implementation information (for example, start time, end time, output setting information, and the like) for the pulsed radiofrequency treatment is stored in the device storage unit 11b. After that, this processing is ended.

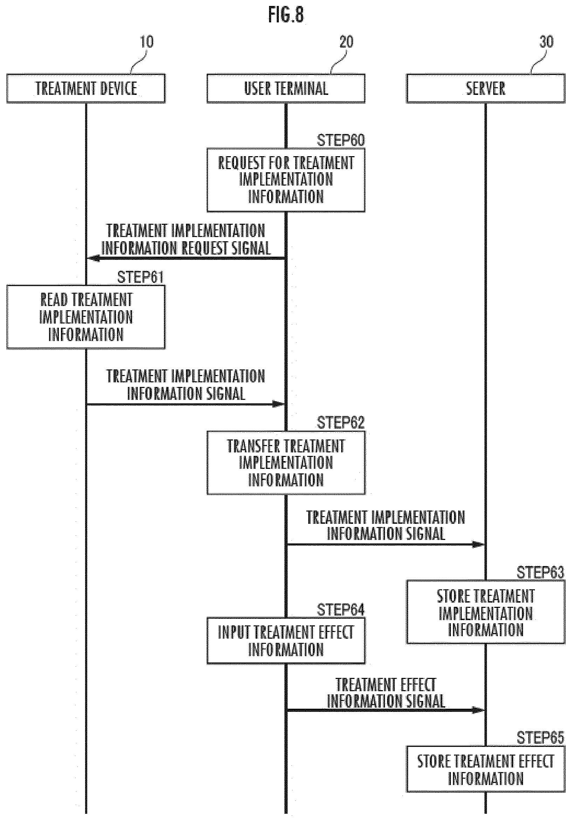

Referring next to FIG. 8, control processing operation executed among the pulsed radiofrequency treatment device 10, the user terminal 20, and the server 30 in the pulsed radiofrequency treatment system will be described.

Here, the control processing operation illustrated in FIG. 8 corresponds to processing after the patient P has received the pulsed radiofrequency treatment, which is executed in a state where the authentication operations of both of the pulsed radiofrequency treatment device 10 and the user terminal 20 are completed therebetween, and the authentication operation of the user terminal 20 is completed between the user terminal 20 and the server 30.

As illustrated in FIG. 8, treatment implementation information request processing is first executed on the user terminal 20 (FIG. 8/STEP60). In this treatment implementation information request processing, treatment implementation information request operation is executed by the patient P operating the user terminal 20. When the treatment implementation information request processing is executed in this way, a treatment implementation information request signal is transmitted from the user terminal 20 to the pulsed radiofrequency treatment device 10.

When this treatment implementation information request signal is received by the pulsed radiofrequency treatment device 10 (more specifically, by the pulse generator 11), treatment implementation information reading processing is executed on the pulsed radiofrequency treatment device 10 (FIG. 8/STEP61).

In this treatment implementation information reading processing, the latest treatment implementation information on the patient P is read from the device storage unit 11b of the pulse generator 11. As described above, this treatment implementation information includes the start time, end time, output setting information, and the like of the pulsed radiofrequency treatment. When the treatment implementation information reading processing is executed in this way, a treatment implementation information signal containing the treatment implementation information is transmitted from the pulsed radiofrequency treatment device 10 to the user terminal 20.

When this treatment implementation information signal is received by the user terminal 20, treatment implementation information transfer processing is executed on the user terminal 20 (FIG. 8/STEP62). In this treatment implementation information transfer processing, the treatment implementation information signal is transferred automatically from the user terminal 20 to the server 30. In this case, it may be so configured that the treatment implementation information signal is transferred from the user terminal 20 to the server 30 by the patient P operating the user terminal 20.

When this treatment implementation information signal is received by the server 30, treatment implementation information storage processing is executed (FIG. 8/STEP63). In this treatment implementation information storage processing, treatment implementation information contained in the treatment implementation information signal is associated with the patient ID of the patient P and the setting information, and stored in the server storage unit 30b as the database of the patient P.

Further, treatment effect information input processing (FIG. 8/STEP64) may be executed on the user terminal 20. In this treatment effect information input processing, the patient P operates the user terminal 20 to input, as treatment effect information, what the patient himself or herself felt about the effect of the pulsed radiofrequency treatment.

When this treatment effect information signal is received by the server 30, treatment effect information storage processing is executed (FIG. 8/STEP65). In this treatment effect information storage processing, treatment effect information contained in the treatment effect information signal is associated with the patient ID of the patient P, the setting information, and the treatment implementation information, and stored in the server storage unit 30b as the database of the patient P.

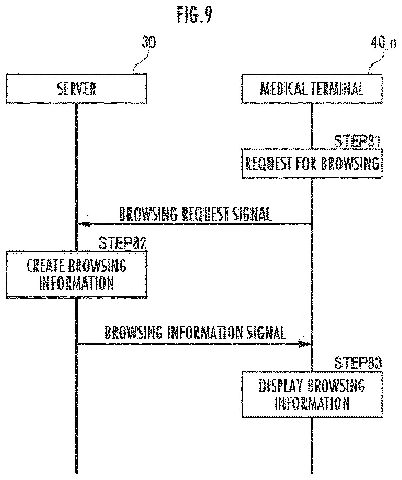

Referring next to FIG. 9, control processing operation executed between the medical terminal 40 and the server 30 in the pulsed radiofrequency treatment system will be described.

This control processing operation illustrated in FIG. 9 is such that a doctor D_n other than the attending doctor D_1 is to access the database inside the server 30 through the medical terminal 40_n, which is executed in a state where the authentication operation of the medical terminal 40_n is completed between the medical terminal 40_n and the server 30.

As illustrated in FIG. 9, browsing request processing is first executed on the medical terminal 40_n (FIG. 9/STEP81). In this browsing processing, the doctor D operates the input interface 40c to input a database name of a pulsed radiofrequency treatment method in such a state that browsing software (for example, a browser) is started up on the medical terminal 40_n. Thus, a browsing request signal including the database name is transmitted from the medical terminal 40_n to the server 30.

When this browsing request signal is received by the server 30, browsing information creation processing is executed on the server 30 (FIG. 9/STEP82). In this browsing information creation processing, the database inside the server 30 is referred to so that browsing information is created based on the database name contained in the browsing request signal.

This browsing information is created to include progress information on pulsed radiofrequency treatments given to many patients. In this progress information, the treatment implementation information and the setting information described above are included, and in addition to these, the treatment effect information described above may also be included. When the browsing information is created in this way, the browsing information signal containing this browsing information is transmitted from the server 30 to the medical terminal 40_n.

When this browsing information signal is received by the medical terminal 40_n, browsing information display processing is executed on the medical terminal 40_n (FIG. 9/STEP83). In this browsing information display processing, the browsing information contained in the browsing information signal is displayed on the display 40b of the medical terminal 40_n.

Thus, the doctor D_n can refer to this browsing information while referencing the progress information on pulsed radiofrequency treatments given to the patient P so far to decide on a treatment plan and the like for patients of the doctor D_n.

Note that, even when the doctor D_n executes own ID authentication with the server 30 using any one of medical terminals 40_1 to 40_n-1 instead of the medical terminal 40_n, control processing operation similar to the control processing operation in FIG. 9 is executable. Further, any one of doctors D_2 to D_n-1 other than the doctor D_n can execute control processing operation similar to the control processing operation in FIG. 9 using any one of medical terminals 40_2 to 40_n-1, respectively.

As described above, according to the pulsed radiofrequency treatment device 10 of the present embodiment, the setting information signal containing the setting information set by the attending doctor D_1 is received by the device communication unit 11d through the user terminal 20, and the setting information is stored in the device storage unit 11b. Then, when treatment start operation is performed by the patient P, the device control unit 11a controls the output state of the radiofrequency pulse current by the pulse generation unit 11e based on the setting information stored in the device storage unit 11b.

Thus, since the pulsed radiofrequency treatment is performed based on the setting information contained in the setting information signal received by the device communication unit 11d of the pulsed radiofrequency treatment device 10, the patient P can receive the pulsed radiofrequency treatment to obtain the optimum treatment effect based on the treatment details set by the attending doctor D_1 in a state of being at home or the like without visiting a medical institution. Therefore, the burden on the patient P can be reduced and convenience can be improved.

Further, in the user terminal 20 of the present embodiment, when the setting information signal is received from the attending doctor terminal 40_1, the setting information signal is transmitted to the device communication unit 11d of the pulsed radiofrequency treatment device 10, and the information on the set size of the electrode pad 12 and the mounting position of the electrode pad 12 onto the patient P is displayed on the terminal display 20c. Therefore, the patient P can recognize the set size of the electrode pad 12 and the mounting position of the electrode pad 12 onto the patient P, and hence the patient P can mount the electrode pad 12 with the right size in a proper affected area.

Further, in the pulsed radiofrequency treatment device 10, when the size of the electrode pad 12 connected to the pulse generator 11 does not match the set size in the setting information, the device display 11c is controlled by the device control unit 11a to display error information indicative of a discrepancy between the size and the set size, and even if the treatment start operation is performed by the patient P, the output of the radiofrequency pulse current from the pulse generation unit 11e will be stopped. Therefore, when the size of the electrode pad 12 mounted on the patient P is wrong, the patient P can recognize that fact and avoid the pulsed radiofrequency treatment being performed in a state where the electrode pad 12 with the wrong size is used.

Further, according to the pulsed radiofrequency treatment system 1 of the present embodiment, the attending doctor terminal 40_1 is operated by the attending doctor D_1 to set the setting information, and when the setting information signal form the attending doctor terminal 40_1 is received by the server 30, the setting information signal is transmitted from the server 30 to the user terminal 20. Further, in the pulsed radiofrequency treatment device 10, at the time when one pulsed radiofrequency treatment is completed, the treatment implementation information signal representing the implementation status of the pulsed radiofrequency treatment is transmitted from the device communication unit 11d to the user terminal 20, and the treatment implementation information signal is transmitted by the user terminal 20 to the server 30.

Then, in the server 30, the setting information from the attending doctor terminal 40_1 and the treatment implementation information from the user terminal 20 are stored in the server storage unit 30b as the database. Therefore, the database obtained by turning the setting information by the attending doctor D_1 and the treatment implementation information on the patient P into data in chronological order can be created inside the server 30. Further, the attending doctor D_1 can access the database through the attending doctor terminal 40_1 to grasp the progress of the pulsed radiofrequency treatment for the patient P.

Further, as described above, when the database contains the treatment effect information from the user terminal 20, the attending doctor D_1 can grasp the feeling of the treatment effect on the patient P in addition to the progress of the pulsed radiofrequency treatment for the patient P, thus enabling the form of treatment for the patient P to be set appropriately. As a result, the effect of the pulsed radiofrequency treatment on the patient P can further be improved.

Further, any doctor D_n other than the attending doctor D_1 can refer to data other than information that identifies the patient P from the database stored in the server storage unit 30b by using the medical terminal 40_n. Therefore, the doctor D_n can improve the knowledge of the pulsed radiofrequency treatment, and can use it as a reference when the doctor D_n himself or herself performs the pulsed radiofrequency treatment.

Note that the embodiment is an example of using the device display 11c as the output interface, but the output interface of the present invention is not limited to this example, and it is fine as long as the output interface outputs information. For example, a speaker may also be provided in the pulse generator 11 as an output interface instead of or in addition to the device display 11c. In this case, it can be configured that, when the electrode pad 12 is inappropriate, error voice is output from the speaker as error information.

Further, the embodiment is an example of using the smartphone-type user terminal 20 as the user terminal, but instead of this, a tablet personal computer, a laptop personal computer, or a desktop personal computer may also be used as the user terminal.

Further, the embodiment is an example of using the terminal display 20c as the terminal output interface, but a speaker of the user terminal 20 may be used as the terminal output interface instead of or in addition to the terminal display 20c. In this case, it can be configured that audio data on the set size and mounting position of the electrode pad 12 is output from the speaker of the user terminal 20.

Further, in the pulsed radiofrequency treatment system 1 of the embodiment, the user terminal 20 may be so omitted that the pulsed radiofrequency treatment device 10 performs direct communication with the server 30 through the communication network 2.

In such a case, it can be configured that the pulse generator 11 of the pulsed radiofrequency treatment device 10 includes a communication device such as a wireless communication device or a LAN communication device to access the communication network 2 directly, and a touch panel display operable by the patient P or the like, and that various control processing in FIG. 6 and FIG. 8 executed by the user terminal 20 of the embodiment is executed by the pulse generator 11.

For example, it can be configured that, when the setting information signal from the server 30 is received by the device communication unit 11d of the pulse generator 11, the information on the set size and mounting position of the electrode pad 12 is displayed on the device display 11c.

### Description of Reference Numerals

- 1: pulsed radiofrequency treatment system
- 2: communication network
- 10: pulsed radiofrequency treatment device
- 11: pulse generator
- 11a: device control unit
- 11b: device storage unit (storage unit)
- 11c: device display (operation unit; output interface)
- 11d: device communication unit (communication unit)
- 11e: pulse generation unit
- 12: electrode pad
- 20: user terminal
- 20c: terminal display (terminal output interface)
- 30: server
- 30b: server storage unit
- 40_1: attending doctor terminal (first medical terminal)
- 40_n: medical terminal (second medical terminal)
- P: patient (person to be treated)
- D_1: attending doctor (therapist)

## Claims

1. A pulsed radiofrequency treatment device comprising:
a pulse generation unit which outputs a radiofrequency pulse current for giving pulsed radiofrequency treatment to a person to be treated;
an electrode pad connected to the pulse generation unit to supply the radiofrequency pulse current to an affected area;
a communication unit which receives, through a communication network, setting information including a set value of the radiofrequency pulse current;
a storage unit which stores the setting information received by the communication unit;
an operation unit for accepting a treatment start operation; and
a control unit which controls an output state of the radiofrequency pulse current by the pulse generation unit based on the setting information stored in the storage unit when the operation unit accepts the treatment start operation.

2. The pulsed radiofrequency treatment device according to Claim 1, further comprising
an output interface which outputs information, wherein
the electrode pad is composed of electrode pads with a plurality of sizes detachable from the pulse generation unit,
the setting information further includes information on a set size of the electrode pad in addition to the set value of the radiofrequency pulse current, and
the control unit controls the output interface to output error information representing a discrepancy between a size of the electrode pad connected to the pulse generation unit and the set size when the size does not match the set size in the setting information, and even when the treatment start operation is performed, the control unit stops output of the radiofrequency pulse current from the pulse generation unit.

3. A pulsed radiofrequency treatment system comprising:
the pulsed radiofrequency treatment device according to Claim 1 or 2;
a server having a server storage unit for storing a database;
a first medical terminal configured to be able to communicate with the server to set the setting information; and
a user terminal configured to be able to communicate with the communication unit of the pulsed radiofrequency treatment device and the server, wherein
at the time when one pulsed radiofrequency treatment is completed, the control unit controls the communication unit to transmit, to the user terminal, treatment implementation information representing implementation status of the pulsed radiofrequency treatment,
the user terminal is configured to be able to transmit, to the server, the treatment implementation information received from the communication unit,
the server is configured to transmit, to the user terminal, the setting information when receiving the setting information from the first medical terminal, and to store the setting information and the treatment implementation information from the user terminal in the server storage unit as the database, and
the first medical terminal is configured to be accessible to the database stored in the server storage unit by communicating with the server.

4. The pulsed radiofrequency treatment system according to claim 3, wherein
the setting information includes information on a mounting position of the electrode pad onto the person to be treated in addition to the set value of the radiofrequency pulse current, and
the user terminal has a terminal output interface to output the information included in the setting information on the mounting position of the electrode pad onto the person to be treated when receiving the setting information from the server.

5. The pulsed radiofrequency treatment system according to claim 3, further comprising
a second medical terminal configured to be able to reference data other than information that identifies the person to be treated from the database stored in the server storage unit by communicating with the server.
